# EUROPEAN PATENT APPLICATION

(11) **EP 4 049 667 A1**
(43) Date of publication of application: **31.08.2022**
(21) Application number: 20878885.1
(22) Date of filing: 20.10.2020
(51) Int. Cl.: A61K 35/747, A23L 33/135, A61K 35/74, A61K 35/745, A61P 3/06, A61P 13/12, C12N 1/20

(54) **UREMIC TOXIN REDUCING AGENT**

(30) Priority: 21.10.2019 JP 2019191754
(71) Applicant: Biofermin Pharmaceutical Co., Ltd., Kobe-shi, Hyogo 650-0021 (JP); Tohoku University, Sendai-shi, Miyagi 980-8577 (JP)
(72) Inventor: MAEDA Ayako, Kobe-shi, Hyogo 651-2242 (JP); TANAKA Yoshiki, Kobe-shi, Hyogo 651-2242 (JP); NAKAJIMA Shunji, Kobe-shi, Hyogo 651-2242 (JP); ABE Takaaki, Sendai-shi, Miyagi 980-8577 (JP)
(74) Representative: Lederer & Keller Patentanwälte Partnerschaft mbB
(86) International application number: PCT/JP2020/039359
(87) International publication number: WO 2021/079869

(57) **Abstract**

A problem to be solved by the present invention is to provide a uremic toxin reducing agent, a total cholesterol reducing agent and a triglyceride reducing agent. The problem is solved by providing a uremic toxin reducing agent, a total cholesterol reducing agent and a triglyceride reducing agent, wherein each agent comprises *Lactobacillus* bacteria, *Bifidobacterium* bacteria or *Bacteroides* bacteria.

## Description

### TECHNICAL FIELD

The present invention relates to a uremic toxin reducing agent, a total cholesterol reducing agent for a subject with reduced kidney function and/or a triglyceride reducing agent for a subject with reduced kidney function, wherein each agent comprises (a) one or more species of *Lactobacillus* bacteria selected from the group consisting of *Lactobacillus gasseri, Lactobacillus johnsonii* and *Lactobacillus casei,* (b) one or more species of *Bifidobacterium* bacteria selected from the group consisting of *Bifidobacterium longum, Bifidobacterium bifidum, Bifidobacterium breve* and *Bifidobacterium infantis,* or (c) one or more species of *Bacteroides* bacteria.

### BACKGROUND ART

Patent literature 1 discloses removal of uremic toxins using a composition containing *Lactobacillus plantarum, Lactobacillus paracasei* or *Streptococcus thermophilus.* However, it has not been reported that *Lactobacillus* bacteria other than these strains, *Bifidobacterium* bacteria and *Bacteroides* bacteria have the effect of reducing uremic toxin levels, the effect of reducing total cholesterol levels in a subject with reduced kidney function and/or the effect of reducing triglyceride levels in a subject with reduced kidney function.

### CITATION LIST

### PATENT LITERATURE

Patent literature 1: JP 2014-133731 A

### SUMMARY OF INVENTION

### TECHNICAL PROBLEM

An object of the present invention is to provide a uremic toxin reducing agent comprising a specific species of bacteria, in particular, *Lactobacillus* bacteria, *Bifidobacterium* bacteria or *Bacteroides* bacteria. Another object of the present invention is to provide a total cholesterol reducing agent for a subject with reduced kidney function and/or a triglyceride reducing agent for a subject with reduced kidney function, wherein each agent comprises any one of the bacteria as described above.

### SOLUTION TO PROBLEM

The inventors conducted extensive studies to solve the above problems and found that an agent comprising (a) one or more species of *Lactobacillus* bacteria selected from the group consisting of *Lactobacillus gasseri, Lactobacillus johnsonii* and *Lactobacillus casei,* (b) one or more species of *Bifidobacterium* bacteria selected from the group consisting of *Bifidobacterium longum, Bifidobacterium bifidum, Bifidobacterium breve* and *Bifidobacterium infantis,* or (c) one or more species of *Bacteroides* bacteria is capable of reducing uremic toxins, and is also capable of reducing total cholesterol levels and/or triglyceride levels in a subject with reduced kidney function. The inventors carried out further studies based on the findings and completed the present invention.

That is, the present invention was made to solve the above problems and includes the following.
(1) A uremic toxin reducing agent comprising any one of the following bacteria (a) to (c):
   (a) one or more species of *Lactobacillus* bacteria selected from the group consisting of *Lactobacillus gasseri, Lactobacillus johnsonii* and *Lactobacillus casei,*
   (b) one or more species of *Bifidobacterium* bacteria selected from the group consisting of *Bifidobacterium longum, Bifidobacterium bifidum, Bifidobacterium breve* and *Bifidobacterium infantis,* or
   (c) one or more species of *Bacteroides* bacteria.
(2) The uremic toxin reducing agent according to the above (1), wherein the one or more species of *Lactobacillus* bacteria are *Lactobacillus gasseri* KD2093 (Accession No. NITE BP-02913).
(3) The uremic toxin reducing agent according to the above (1), wherein the one or more species of *Bifidobacterium* bacteria are *Bifidobacterium bifidum* G9-1 (Accession No. NITE BP-817).
(4) The uremic toxin reducing agent according to the above (1), wherein the one or more species of *Bacteroides* bacteria are *Bacteroides thetaiotaomicron.*
(5) The uremic toxin reducing agent according to any one of the above (1) to (4), wherein a uremic toxin to be reduced is one or more selected from the group consisting of blood urea nitrogen (BUN), 1-methyladenosine (m1A), trimethylamine-N-oxide (TMAO), phenyl sulfate (PS), indoxyl sulfate (IS) and 4-ethylphenyl sulfate (4-EPS).
(6) The uremic toxin reducing agent according to any one of the above (1) to (5), wherein the agent further reduces a creatinine level.
(7) The uremic toxin reducing agent according to any one of the above (1) to (6), wherein the agent further increases a hematocrit level and/or a hemoglobin level.
(8) A method for producing the uremic toxin reducing agent according to any one of the above (1) to (7), comprising culturing any one of the bacteria as described in the above (1), and collecting a culture product.
(9) Use of any one of the bacteria as described in the above (1) in the production of a uremic toxin reducing agent.
(10) A total cholesterol reducing agent for a subject with reduced kidney function, comprising any one of the following bacteria (a) to (c):
   (a) one or more species of *Lactobacillus* bacteria selected from the group consisting of *Lactobacillus gasseri, Lactobacillus johnsonii* and *Lactobacillus casei,*
   (b) one or more species of *Bifidobacterium* bacteria selected from the group consisting of *Bifidobacterium longum, Bifidobacterium bifidum, Bifidobacterium breve* and *Bifidobacterium infantis,* or
   (c) one or more species of *Bacteroides* bacteria.
(11) A triglyceride reducing agent for a subject with reduced kidney function, comprising any one of the following bacteria
   (a) to (c):
   (a) one or more species of *Lactobacillus* bacteria selected from the group consisting of *Lactobacillus gasseri, Lactobacillus johnsonii* and *Lactobacillus casei,*
   (b) one or more species of *Bifidobacterium* bacteria selected from the group consisting of *Bifidobacterium longum, Bifidobacterium bifidum, Bifidobacterium breve* and *Bifidobacterium infantis,* or
   (c) one or more species of *Bacteroides* bacteria.

### ADVANTAGEOUS EFFECTS OF INVENTION

The present invention provides a uremic toxin reducing agent, a total cholesterol reducing agent for a subject with reduced kidney function and/or a triglyceride reducing agent for a subject with reduced kidney function, wherein each agent comprises (a) one or more species of *Lactobacillus* bacteria selected from the group consisting of *Lactobacillus gasseri, Lactobacillus johnsonii* and *Lactobacillus casei,* (b) one or more species of *Bifidobacterium* bacteria selected from the group consisting of *Bifidobacterium longum, Bifidobacterium bifidum, Bifidobacterium breve* and *Bifidobacterium infantis,* or (c) one or more species of *Bacteroides* bacteria. The agents of the present invention can be used for industrial fermentation to efficiently produce the uremic toxin reducing agent or the total cholesterol and/or triglyceride reducing agent for a subject with reduced kidney function in an industrially advantageous manner. Alternatively, the agents of the present invention can be orally administered to a living body, such as a human or a mammal, to serve as a highly active uremic toxin reducing agent or a highly active total cholesterol and/or triglyceride reducing agent for a subject with reduced kidney function.

### BRIEF DESCRIPTION OF DRAWINGS

Fig. 1 is a schematic view of a spray dryer.
Fig. 2 shows a feeding schedule for mice. In the figure, "RF" represents renal failure, and "dw" represents distilled water. The same abbreviations are used in the subsequent figures.
Fig. 3 shows that the bacteria of the present invention reduce the increased level of a uremic toxin (blood urea nitrogen (BUN)) in the blood induced by adenine-induced renal failure and increase the reduced levels of hemoglobin (Hb) and hematocrit (Hct) induced by adenine-induced renal failure.
Fig. 4 shows that *Lactobacillus gasseri* KD2093 reduces the increased levels of creatinine, 1-methyladenosine (m1A), trimethylamine-N-oxide (TMAO), phenyl sulfate (PS) and indoxyl sulfate (IS).
Fig. 5 shows that *Bifidobacterium bifidum* G9-1 reduces the increased levels of creatinine, 1-methyladenosine, trimethylamine-N-oxide, phenyl sulfate and indoxyl sulfate.
Fig. 6 shows that *Bacteroides thetaiotaomicron* 6030 reduces the increased levels of creatinine, 1-methyladenosine, trimethylamine-N-oxide, phenyl sulfate and indoxyl sulfate.
Fig. 7 shows that dead bacterial cells of *Lactobacillus gasseri* KD2093 reduce the increased levels of total cholesterol (T-Cho or TC) and triglyceride (TG) in a renal failure model.

### DESCRIPTION OF EMBODIMENTS

### (a) Lactobacillus bacteria

(a) *Lactobacillus* bacteria contained in the agent of the present invention are preferably one or more species selected from the group consisting of *Lactobacillus acidophilus, L. gasseri, L. johnsonii, L. paracasei* subsp. *paracasei, L. reuteri, L. rhamnosus, L. salivarius, L. brevis, L. crispatus, L. delbrueckii* (*L. delbrueckii* subsp. *delbrueckii*), *L. bulgaricus* (*L. delbrueckii* subsp. *bulgaricus*), *L. lactis* (*L. delbrueckii* subsp. *lactis*), *L. fermentum, L. casei,* etc. Among these bacterial species, the agent preferably comprises one or more species selected from the group consisting of *Lactobacillus gasseri, Lactobacillus johnsonii* and *Lactobacillus casei.* More preferably, the agent comprises *Lactobacillus acidophilus* or *L. gasseri,* particularly preferably *L. gasseri,* particularly preferably *L. gasseri* KD2093 (Accession No.: NITE BP-02913).

When the agent of the present invention is in the form of, for example, a liquid, the amount of the *Lactobacillus* bacteria contained in the agent may be, for example, but is not limited to, about 10⁴ to 10¹⁰ cfu/mL, preferably about 10⁶ to 10⁹ cfu/mL. When the agent is in the form of a solid, the amount of the *Lactobacillus* bacteria contained in the agent may be, for example, but is not limited to, 10⁵ to 10¹⁰ cfu/g based on the total weight of the agent, preferably 10⁶ to 10⁹ cfu/g based on the total weight of the agent.

### (b) Bifidobacterium bacteria

(b) *Bifidobacterium* bacteria contained in the agent of the present invention are one or more species selected from the group consisting of *Bifidobacterium longum, Bifidobacterium bifidum, Bifidobacterium breve, Bifidobacterium infantis, Bifidobacterium animalis* (*Bifidobacterium animalis* subsp. *animalis*), *Bifidobacterium lactis* (*Bifidobacterium animalis* subsp. *lactis*), etc. More preferably, the agent comprises *Bifidobacterium longum* or *Bifidobacterium bifidum,* particularly preferably *Bifidobacterium bifidum,* most preferably *Bifidobacterium bifidum* G9-1.

The agent of the present invention may further comprise *Bifidobacterium* bacteria other than those described in the above (b). For example, the agent may further comprise *Bifidobacterium adolescentis, Bifidobacterium pseudolongum,* or *Bifidobacterium thermophilum.* Preferably, the agent further comprises *Bifidobacterium pseudolongum* or *Bifidobacterium thermophilum.* Said other *Bifidobacterium* bacteria are not limited to these.

When the agent of the present invention is in the form of, for example, a liquid, the amount of the *Bifidobacterium* bacteria contained in the agent may be, for example, but is not limited to, about 10⁴ to 10¹⁰ cfu/mL, preferably about 10⁶ to 10⁹ cfu/mL. When the agent is in the form of a solid, the amount of the *Bifidobacterium longum* bacteria contained in the agent may be, for example, but is not limited to, 10⁵ to 10¹⁰ cfu/g based on the total weight of the agent, preferably 10⁶ to 10⁹ cfu/g based on the total weight of the agent.

### (c) Bacteroides bacteria

(c) *Bacteroides* bacteria contained in the agent of the present invention are preferably one or more species selected from the group consisting of *Bacteroides thetaiotaomicron, Bacteroides fragilis, Bacteroides distasonis, Bacteroides ovatus, Bacteroides uniformis, Bacteroides vulgatus, Bacteroides oleiciplenus, Bacteroides clarus, Bacteroides fluxus, Bacteroides dorei, Bacteroides faecis, Bacteroides intestinalis, Bacteroides massiliensis, Bacteroides merdae,* etc. More preferably, the agent comprises *Bacteroides thetaiotaomicron, Bacteroides uniformis* or *Bacteroides vulgatus,* particularly preferably *Bacteroides thetaiotaomicron,* most preferably *Bacteroides thetaiotaomicron* 6030.

When the agent of the present invention is in the form of, for example, a liquid, the amount of the *Bacteroides* bacteria contained in the agent may be, for example, but is not limited to, about 10⁴ to 10¹⁰ cfu/mL, preferably about 10⁶ to 10⁹ cfu/mL. When the agent is in the form of a solid, the amount of the *Bacteroides* bacteria contained in the agent may be, for example, but is not limited to, 10⁵ to 10¹⁰ cfu/g based on the total weight of the agent, preferably 10⁶ to 10⁹ cfu/g based on the total weight of the agent.

The agent may comprise a single bacterial species as described in the above (a), (b) or (c) or may comprise a plurality of bacterial species as described in the above (a), (b) or (c) . The uremic toxin reducing agent, the total cholesterol reducing agent for a subject with reduced kidney function and/or the triglyceride reducing agent for a subject with reduced kidney function according to the present invention preferably comprises one to five bacterial species as described in the (a), (b) or (c), more preferably comprises one to three bacterial species as described in the (a), (b) or (c), and particularly preferably comprises one or two bacterial species as described in the (a), (b) or (c).

The agent of the present invention may further comprise another bacterial species in addition to those listed in the above (a), (b) or (c). Said another bacterial species preferably includes, for example, but is not limited to, *Leuconostoc* spp. such as *Leuconostoc mesenteroides; Streptococcus* spp. such as *Streptococcus* (*Enterococcus*) *faecalis, Streptococcus (Enterococcus) faecium, Streptococcus* (*Enterococcus*) *hirae* and *Streptococcus thermophilus* (according to the current taxonomy, these bacterial species belong to *Enterococcus* spp.); *Lactococcus* spp. such as *Lactococcus lactis* and *L. cremoris; Tetragenococcus* spp. such as *Tetragenococcus halophilus; Pediococcus* spp. such as *Pediococcus acidilactici* and *P. pentosaceus; Oenococcus* spp. such as *Oenococcus oeni;* etc.

The bacterial species herein are classified according to the current taxonomy, but *Streptococcus* spp. herein may include both of *Streptococcus* spp. and *Enterococcus* spp. according to the old taxonomy.

The agent of the present invention may further comprise, for example, known beneficial bacteria, such as hay bacillus, amylolytic bacteria and butyric acid bacteria, in addition to those listed in the above (a), (b) or (c). Examples of such beneficial bacteria may include amylolytic bacteria, such as *Bacillus subtilis* (hay bacillus), *Bacillus mesentericus* and *Bacillus polyfermenticus;* spore forming lactic acid bacteria, such as *Bacillus coagulans;* butyric acid bacteria, such as *Bacillus toyoi, Bacillus licheniformis* and *Clostridium butyricum;* and others. These beneficial bacteria are typically non-pathogenic.

The present invention relates to the uremic toxin reducing agent, the total cholesterol reducing agent for a subject with reduced kidney function, and the triglyceride reducing agent for a subject with reduced kidney function, as described above. The uremic toxin reducing agent of the present invention is useful for, for example, prevention or treatment of renal diseases. The "prevention" of renal diseases herein means, for example, prevention of the onset of renal diseases. The "treatment" of renal diseases herein means, for example, remission of the symptoms of renal diseases or complete healing from renal diseases.

The total cholesterol reducing agent and the triglyceride reducing agent for a subject with reduced kidney function are useful for, for example, prevention or treatment of fat-associated diseases. The "prevention" of fat-associated diseases herein means, for example, prevention of the onset of fat-associated diseases. The "treatment" of fat-associated diseases herein means, for example, remission of the symptoms of fat-associated diseases or complete healing from fat-associated diseases.

Renal diseases for which the agent of the present invention is indicated include, for example, but are not limited to, chronic kidney disease, renal failure, chronic pyelonephritis, acute pyelonephritis, chronic glomerulonephritis, advanced acute nephritic syndrome, nephrotic syndrome, nephrosclerosis, interstitial nephritis, diabetic nephropathy, focal glomerulosclerosis, membranous nephropathy, polycystic kidney syndrome, renovascular hypertension, and hypertension syndrome, and secondary renal diseases associated with the above primary diseases . Renal diseases in a broad sense include hyperphosphatemia, hyperkalemia, hyperuricemia, kidney injury with hyponatremia, hypernatremia, etc. associated with chronic kidney diseases.

Fat-associated diseases for which the agent of the present invention is indicated include, for example, but are not limited to, metabolic syndrome, nonalcoholic fatty liver disease (NAFLD) (including nonalcoholic steatohepatitis (NASH)), hyperlipemia, hyperuricemia, diabetes mellitus, hypertension, cerebral infarction, gout, sleep apnea syndrome, obesity hypoventilation syndrome, menstrual disorders, and pregnancy complications. Metabolic syndrome is a condition that includes a cluster of diseases and abnormalities. Examples of the diseases and abnormalities include, but are not limited to, obesity (for example, lipid metabolic abnormalities, fatty liver, etc.), carbohydrate metabolic abnormalities, abnormal insulin resistance, heart diseases such as angina pectoris and myocardial infarction, arteriosclerotic diseases (for example, cerebral infarction, arteriosclerosis obliterans, etc.), etc. Examples of the diseases that are developed or affected in association with fat-related diseases include cirrhosis, liver cancer, etc.

The dosage of the bacteria of the present invention to an animal including humans is preferably about 1 × 10³ to 1 × 10¹¹ cells per dose per adult, more preferably about 1 × 10⁶ to 1 × 10¹¹ cells per dose per adult, further more preferably about 1 × 10⁸ to 1 × 10¹¹ cells per dose per adult.

The dosage of a processed product of the bacteria of the present invention to an animal including humans in terms of the number of processed bacterial cells is preferably about 1 × 10³ to 1 × 10¹⁴ cells per dose per adult, more preferably about 1 × 10⁶ to 1 × 10¹⁴ cells per dose per adult, further more preferably about 1 × 10⁸ to 1 × 10¹⁴ cells per dose per adult.

The frequency of administration of the agent of the present invention will depend on, for example, the subject for administration such as rats, the route of administration such as oral administration route, the dosage form such as a liquid, or other factors, but the agent of the present invention may be administered, for example, once to five times a day, or may be administered once to five times a week, or may be administered once to five times a month.

The duration of administration of the agent of the present invention will depend on, for example, the subject for administration such as rats, the route of administration such as oral administration route, the dosage form such as a liquid, the frequency of administration, or other factors, but the agent of the present invention may be administered, for example, for one to six days, or may be administered for one week to four weeks, or may be administered for one month to 12 months. Alternatively, for example, a composition containing the agent may be continuously administered.

A subject for administration of the agent of the present invention may be, for example, animals, including, for example, humans, rats, mice, rabbits, sheep, pigs, cows, cats, dogs, monkeys, etc.

A subject for administration of a prophylactic or therapeutic agent for a renal disease according to the present invention is preferably an animal other than dogs or cats, and includes, for example, humans, rats, mice, rabbits, sheep, pigs, cows, monkeys, etc.

### Acquisition of bacterial cells

The *Lactobacillus* bacteria, the *Bifidobacterium* bacteria, the *Bacteroides* bacteria or other beneficial bacteria as described above are easily available from, for example, public organizations such as ATCC (registered trademark) or IFO, the Japan Bifidus Foundation, Patent Microorganisms Depositary of National Institute of Technology and Evaluation (incorporated administrative agency), or other providers. Commercially available bacteria may also be used as appropriate.

For example, *Bifidobacterium bifidum* G9-1 was internationally deposited with Patent Microorganisms Depositary of National Institute of Technology and Evaluation (NPMD)(incorporated administrative agency) (address: #122, 2-5-8 Kazusakamatari, Kisarazu-shi, Chiba 292-0818 Japan) on September 30, 2009 (date of the original deposit: September 17, 2009) under Accession No. NITE BP-817.

For example, *Lactobacillus gasseri* KD2093 was internationally deposited with Patent Microorganisms Depositary of National Institute of Technology and Evaluation (NPMD)(incorporated administrative agency) (address: #122, 2-5-8 Kazusakamatari, Kisarazu-shi, Chiba 292-0818 Japan) on September 10, 2020 (Date of the original deposit: March 12, 2019) under Accession No. NITE BP-02913.

Theses bacteria to be cultured may be from a frozen stock.

### Culture of bacteria

The *Lactobacillus* bacteria, the *Bifidobacterium* bacteria, the *Bacteroides* bacteria or other beneficial bacteria as described above are usually inoculated in a culture medium and cultured. The basic composition of the culture medium for the bacteria may be established based on known culture media for lactic acid bacteria, including, for example, high nutrient growth media for general purpose, such as MRS medium, LBS medium, and Rogosa medium.

A culture medium for anaerobes is also suitable for culture of the bacteria. For example, GAM liquid medium, such as GAM broth and modified GAM broth, is suitable for culture of the bacteria, but the culture medium is not limited thereto.

The culture medium used in the present invention may contain, but not limited to, a carbon source, a nitrogen source, amino acids, vitamins, minerals, animal and plant proteins or an extract or a decomposed product thereof, inorganic salts, buffering agents, surfactants, antibiotics, stabilizers, water or any combination thereof. Such ingredients contained in the culture medium may be commercially available products.

The nitrogen source may be, for example, peptone from animal tissues or plants; an ammonium salt, such as ammonium nitrate, ammonium sulfate, ammonium chloride and ammonium acetate; a hydrated ammonium salt; ammonia; etc. The peptone is preferably, but not limited to, for example, soy peptone, proteose peptone, casein peptone, heart peptone, meat peptone, etc. The amount of the nitrogen source contained in the culture medium may be, but is not limited to, for example, 0.1 to 1% by weight or 0.1 to 0.5% by weight based on the total weight of the culture medium.

The carbon source may be, for example, a monosaccharide, a disaccharide, a trisaccharide, a tetrasaccharide, an oligosaccharide, a polysaccharide, etc. Examples of the monosaccharide include glucose, xylose, arabinose, mannose, galactose or any combination thereof. Examples of the disaccharide include maltose, cellobiose, trehalose, sucrose, lactulose, lactose or any combination thereof. The amount of the carbon source contained in the culture medium may be, but is not limited to, for example, 0.1 to 1% by weight or 0.1 to 0.5% by weight based on the total weight of the culture medium.

The culture medium used in the present invention preferably contains a growth factor such as amino acids and vitamins. Examples of the amino acids include, but are not limited to, alanine, cysteine, aspartic acid, glutamic acid, phenylalanine, glycine, histidine, isoleucine, lysine, leucine, methionine, asparagine, pyrrolysine, proline, glutamine, arginine, serine, threonine, selenocysteine, valine, tryptophan, tyrosine, salts thereof, and any combination thereof. These amino acids are usually L-form amino acids. The amount of the amino acids contained in the culture medium may be, but is not limited to, for example, 0.01 to 0.1% by weight or 0.01 to 0.05% by weight based on the total weight of the culture medium.

The vitamins are preferably, but not limited to, vitamins A, B, C, D, E and K, or a derivative thereof, or a salt thereof, biotin, riboflavin, thiamin, or any combination thereof. The amount of the vitamins contained in the culture medium may be, but is not limited to, for example, 0.01 to 0.1% by weight or 0.01 to 0.05% by weight based on the total weight of the culture medium.

The minerals are preferably, but not limited to, magnesium, potassium, calcium, phosphorus, zinc, iron, etc. The amount of the minerals contained in the culture medium may be, but is not limited to, for example, 0.01 to 0.1% by weight or 0.01 to 0.05% by weight based on the total weight of the culture medium.

The animal and plant proteins or an extract or a decomposed product thereof is preferably, for example, a plant extract, a meat extract, a liver extract or a yeast extract. The amount of such an extract or a decomposed product thereof contained in the culture medium may be, but is not limited to, for example, 0.1 to 1% by weight or 0.1 to 0.5% by weight based on the total weight of the culture medium.

The inorganic salts may be, but are not limited to, for example, a phosphate, sodium chloride, sodium nitrate, potassium nitrate, manganese sulfate hydrate, magnesium sulfate hydrate, etc. The amount of the inorganic salts contained in the culture medium may be, but is not limited to, for example, 0.01 to 0.1% by weight or 0.01 to 0.05% by weight based on the total weight of the culture medium.

The buffering agents may be, but are not limited to, for example, PBS, HBSS, HEPES, HANKS, etc. The surfactants are preferably, but not limited to, for example, polysorbates, such as polysorbate 20, polysorbate 60, polysorbate 65, and polysorbate 80, macrogol, sodium lauryl sulfate, etc. The antibiotics may be, but are not limited to, for example, mycin antibiotics, such as penicillin, streptomycin and kanamycin.

The culture medium may also contain other ingredients or additives, including, but not limited to, carbonates, bicarbonates, albumin, insulin, transferrin, selenium, hormones, cytokines, L-cysteine hydrochloride, sodium thioglycolate, hemin, soluble starch, digested serum powder, vitamins, short-chain fatty acids, etc.

The ingredients as described above can be mixed together, and heat-sterilized in a high pressure steam sterilization pot to prepare the culture medium.

The pH of the culture medium is preferably neutral (for example, at a pH of 6 to 8, or at a pH of 7 to 8). A pH adjuster known in the art or the buffer agents as described above can be used to neutralize the culture medium.

When the *Lactobacillus* bacteria, the *Bifidobacterium* bacteria, the *Bacteroides* bacteria or other beneficial bacteria are inoculated in a culture medium or into an animal body, the amount of the bacteria relative to the amount of the culture medium may be, but is not limited to, for example, 10¹ to 10⁸ cfu/mL or 10⁵ to 10⁸ cfu/mL. The culture medium for inoculation (seed culture) may be the same as or different from the culture medium for growth (main culture).

The culture temperature is preferably, for example, 25 to 45°C, more preferably 36 to 38°C. The culture time is preferably, for example, 4 to 72 hours, more preferably 12 to 24 hours. The *Bifidobacterium* bacteria easily grow under the culture temperature and/or for the culture time.

An anaerobic box or an anaerobic chamber may be used to culture the *Bifidobacterium* bacteria under anaerobic conditions. The anaerobic box or the anaerobic chamber may be commercially available products.

The bacteria used in the present invention may be in any form, including, but not limited to, live bacterial cells, wet bacterial cells, dried bacterial cells, or other types of bacterial cells.

A processed product of the bacteria prepared by processing live bacterial cells, wet bacterial cells, dried bacterial cells, or other types of bacterial cells may also be used. The term "processed product of the bacteria" refers to a product prepared by subjecting the lactic acid bacteria to any type of processing. Specific examples of the processed product of the bacteria include a suspension of the bacterial cells disrupted by, e.g., sonication; the culture medium or culture supernatant of the bacterial cells; the solid residues separated from such disrupted cell suspension, culture medium or culture supernatant by solid-liquid separation techniques such as filtration or centrifugation. The processed product of the bacteria also includes a processed solution obtained by removing the cell walls by enzymatic or mechanical techniques; protein complexes (e.g., proteins, lipoproteins, glycoproteins, etc.) or peptide complexes (e.g., peptides, glycopeptides, etc.) prepared by trichloroacetic acid treatment or salting-out process. The processed product of the bacteria further includes a concentrate, a dilution or a dried product of the above processed products. Techniques for collecting bacterial cells from the culture medium to obtain non-processed bacterial cells are well established in the art, and the collection of bacterial cells may be performed in accordance with such techniques. The processed product of the bacteria used in the present invention also includes a further processed product prepared by subjecting a suspension of disrupted (e.g., sonicated) bacterial cells, or the culture medium or culture supernatant of the bacterial cells, or the like to, for example, separation by various types of chromatography, etc.

Dead bacterial cells are also included in the processed product of the bacteria in the present invention. Dead bacterial cells can be prepared by, for example, enzymatic treatment, heat treatment, drug treatment using, e.g., antibiotics, chemical treatment using, e.g., formalin, radiation treatment using, e.g., γ-rays, etc. Such treatment techniques are well established in the art, and the treatment according to the present invention may be performed in accordance with such techniques.

A preferred method for preparing dried bacterial cells or wet bacterial cells will be described below. Bacterial cells collected from the culture medium are dispersed in a solvent to prepare a bacterial cell liquid. The solvent used for dispersion of the bacterial cells to prepare a bacterial cell liquid may be a known solvent commonly used in the art, but is preferably a buffer solution, such as water and PBS. If desired, ethanol or the like may be added to the solvent. The bacterial cell liquid may be a suspension in a solvent, and the solvent may be the same as those described above. When the bacterial cells are suspended in a solvent, a suspending agent such as sodium alginate may be used.

The bacterial cell liquid may further contain an additive commonly used in the art, such as an excipient, a binder, a disintegrant and an antistatic, in a usual amount in accordance with a known technique.

Examples of the excipient include lactose, sucrose, D-mannitol, corn starch, powdered cellulose, calcium hydrogen phosphate and calcium carbonate. Examples of the binder include hydroxypropyl cellulose, polyvinylpyrrolidone and xanthan gum. Examples of the disintegrant include low-substituted hydroxypropyl cellulose, carmellose calcium, partially pregelatinized starch, croscarmellose sodium, crospovidone and carboxy methyl starch. Examples of the antistatic include fine talc powder or no fine talc powder, colloidal silica, processed silica and precipitated silica.

The bacterial cell liquid may be sterilized. Sterilization is preferably performed by, for example, filtering, but may be performed by other known sterilization methods, including, for example, heat sterilization methods, such as steam heat sterilization, dry heat sterilization and high frequency sterilization; gas sterilization methods, such as ethylene oxide gas sterilization and hydrogen peroxide gas sterilization; and radiation sterilization methods, such as gamma radiation sterilization and electron beam radiation sterilization.

The bacterial cell liquid can be subjected to drying in a spray dryer to prepare dried bacterial cells. The spray dryer is preferably equipped with an atomizer capable of forming spray droplets with a single micron size. Spray droplets with a very small particle size will have a large surface area per unit mass, and the spray droplets efficiently contact with warm drying air and improve the productivity.

The term "droplets with a single micron size" refers to spray droplets preferably having a particle size of 1 to 10 µm when rounded to the nearest whole number.

Examples of the spray dryer include a spray dryer equipped with an atomizer, which may be, for example, a rotary atomizer (a rotary disk), a pressure nozzle, or a two-fluid or four-fluid nozzle using the force of a compressed gas.

The spray dryer may be any of the above described types that can form spray droplets with a single micron size, but preferred is a spray dryer equipped with a four-fluid nozzle.

The four-fluid nozzle of the spray dryer preferably has, for example, a structure in which a gas passage is connected with a liquid passage to create a single unit system, and two unit systems are symmetrically disposed about the nozzle edge. The unit systems are each provided with a slope to direct the fluid flow to the nozzle edge.

The spray dryer is preferably equipped with an external mixing atomizer for directing a compressed gas and a liquid from the both sides onto a single collision point at the tip of the nozzle edge. This type of atomizer prevents clogging of the nozzle and allows for spraying for a long period of time.

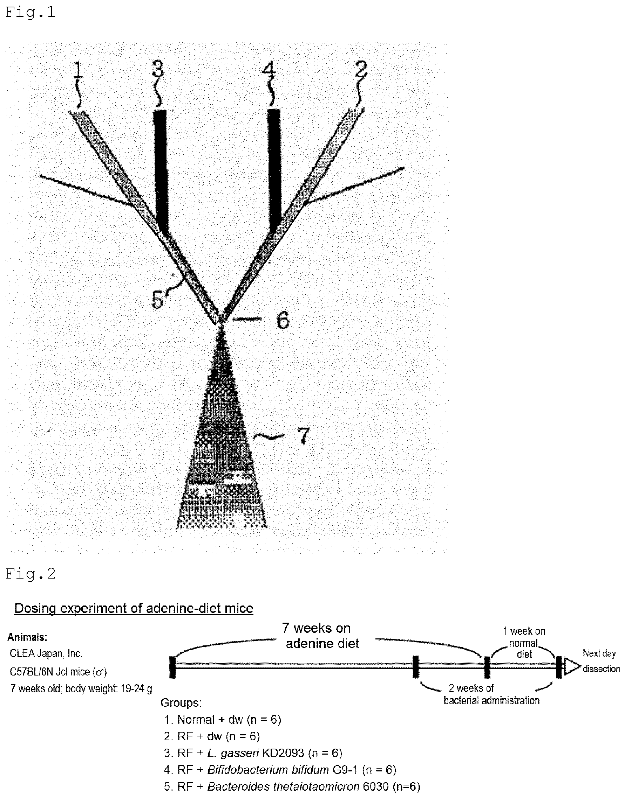

The spray dryer with a four-fluid nozzle will be described in more detail below with reference to Fig. 1. In the nozzle edge of the four-fluid nozzle, the bacterial cell liquid poured through liquid passages 3 and 4 is stretched into a very thin stream on fluid flow surfaces 5 by high-speed gas flows from gas passages 1 and 2. The stretched liquid is then atomized into spray droplets 7 with a single micron size by the shock wave generated at the collision point 6 at the tip of the nozzle edge.

Examples of the compressed gas include inert gas, such as air, carbon dioxide gas, nitrogen gas and argon gas. When easily oxidized materials or other materials are spray-dried, inert gas is preferred, including carbon dioxide gas, nitrogen gas and argon gas. The pressure of the compressed gas is usually about 1 to 15 kgf/cm², preferably about 3 to 8 kgf/cm². The gas flow rate at the nozzle is usually about 1 to 100 L/min, preferably about 10 to 20 L/min, per mm of the nozzle edge.

The spray droplets are then usually contacted with warm drying air in a drying chamber to evaporate the moisture to give dried bacterial cells.

The inlet temperature of the drying chamber is usually about 2 to 400°C, preferably about 5 to 250°C, and more preferably about 5 to 150°C. Even when the inlet temperature is as high as about 200 to 400°C, the temperature in the drying chamber does not become excessively high due to the heat of evaporation of moisture. The death of or damage to live bacteria can be inhibited to some extent by reducing the retention time in the drying chamber.

The outlet temperature is usually about 0 to 120°C, preferably about 5 to 90°C, and more preferably about 5 to 70°C.

The reduction in the particle size of the dried bacterial cells as described above leads to an increase in the percentage of live bacteria, thereby providing a formulation with a high content of live bacteria. In other words, the bacterial cell liquid is preferably sprayed into single micron-sized spray droplets to produce single micron-sized dried bacterial cells. This reduction in the particle size of the spray droplets leads to an increase in the surface area per unit mass of the spray droplets. When the surface area is increased, the spray droplets efficiently contact with warm drying air, and the death of or damage to the bacterial cells due to the heat from warm drying air is inhibited to the best extent possible. As a result, the percentage of live bacteria is increased, and dried bacterial cells containing a large number of live bacteria are provided.

The wet bacterial cells can be obtained by a method known in the art. For example, bacterial cells are collected from the culture medium by centrifugation, washed with phosphate buffer solution, centrifuged again, and frozen for storage.

The agent of the present invention can usually be easily produced by mixing the *Lactobacillus* bacteria, the *Bifidobacterium* bacteria, the *Bacteroides* bacteria and/or other beneficial bacteria with another ingredient. Said another ingredient may be any ingredient that does not impair the effects of the present invention. The agent of the invention may be formulated into a medicine, a quasi-drug, a food or drink, a food additive, a dietary supplement, a feed, etc. Such a medicine containing the agent of the invention is also a preferred embodiment of the present invention.

The food or drink includes health foods, functional foods, foods for specified health use, and foods for sick people. Such a food or drink may be in any form, including, for example, drinks such as refreshing drinks, carbonated drinks, nutritional drinks, fruit juice, lactic drinks, and tea drinks; sweets and bakery products such as drops, candies, gummy candies, chewing gum, chocolate, snacks, biscuits, jellies, jam, cream, pastries, and bread; noodles such as buckwheat noodles, wheat noodles, Chinese noodles, and instant noodles; fishery and livestock products such as ham, sausages, and fish sausages; dairy products such as processed milk and fermented milk; fats, oils and processed foods thereof, such as vegetable oil, oil for deep frying, butter, margarine, mayonnaise, shortening, dressing, and whipped cream; seasonings such as sauce and ketchup; retort pouch foods such as curry, stew, rice-bowl cuisine, and rice soup; and frozen desserts such as ice cream, sherbet, and shaved ice. The food or drink of the present invention also includes foods for special health use, dietary supplements, supplemental foods, functional foods or the like that have health claims based on the concept of reducing uremic toxins, reducing total cholesterol in a subject with reduced kidney function and/or reducing triglyceride in a subject with reduced kidney function. The term "health foods" refers to food compositions for health care, health maintenance, health enhancement or other purposes, and may include those approved as foods with functional claims, and the so-called health foods that have not been approved as foods with functional claims.

The present invention can also provide a composition containing the agent of the present invention. The composition of the present invention may further contain a known additive commonly used in the art, including, but not limited to, for example, water, solvents, pH adjusting agents, moisturizers, flavoring agents, sweeteners, thickeners, flavor improvers, gelling agents, solubilizers, colorants, antiseptics, surfactants, suspending agents, emulsifiers and stabilizers.

The *Lactobacillus* bacteria, the *Bifidobacterium* bacteria, the *Bacteroides* bacteria and other beneficial bacteria are generally anaerobic bacteria and have low tolerance to air and oxygen in a dry state, and are also susceptible to high temperature and moisture. Therefore when these bacteria are formulated into a composition, the bacteria are preferably processed at a low temperature under an inert gas or vacuum.

The agent of the present invention may be administered to a human or a non-human animal. The mode of administration of the agent of the invention may be, but is not limited to, oral administration, parenteral administration (intravenous administration, transdermal administration, topical ocular administration, etc.), or other modes of administration. When the agent is an oral formulation, the dosage form may be a tablet, a capsule, granules, a powder, etc. When the agent is a parenteral formulation, the dosage form may be an insert dosage form, etc. The dosage of the agent can be determined as appropriate depending on the dosage form, the symptoms, age, and body weight of the patient, etc. For example, for oral administration, 0.05 to 5000 mg/kg body weight per day, preferably 0.1 to 2000 mg/kg body weight per day, further preferably 1 to 1000 mg/kg body weight per day, can be administered in a single dose or several divided doses per day, but the dosage is not limited thereto.

### Uremic toxin reducing agent

The "uremic toxins" as referred herein include, for example, but are not limited to, blood urea nitrogen (BUN), 1-methyladenosine (m1A), trimethylamine-N-oxide (TMAO), phenyl sulfate (PS), indoxyl sulfate (IS), creatinine, etc. The uremic toxins herein may also include, for example, trimethylamine, indole, indoleacetic acid, guanidinoacetic acid, paracresol, hippuric acid, furandicarboxylic acid, and homocysteine, and precursors thereof.

The uremic toxin reducing agent of the present invention may be administered not only to reduce the uremic toxins but also to improve hematocrit levels and/or anemia.

In order to examine whether the uremic toxins have been reduced, any known method can be used in the present invention. For example, serum urea nitrogen (BUN) can be measured as later described in Examples to determine whether the uremic toxin is reduced by administration of the bacteria or a processed product thereof of the present invention or the agent of the present invention. For example, when the BUN level is measured and found to be reduced after administration of the bacteria or a processed product thereof or the agent compared with that before the administration, one can determine that the bacteria or a processed product thereof or the agent has uremic toxin reducing effect. The indicators other than BUN as described below may also be used to determine whether the bacteria or a processed product thereof or the agent has uremic toxin reducing effect.

1-methyladenosine (m1A) is a uremic toxin, and is a component specifically found in tRNA. m1A has recently been described as an indicator for the presence of damage of tissue due to oxidative stress. When, for example, the m1A level in a group receiving the bacteria or a processed product thereof of the present invention or the agent of the present invention is lower than that in a group not receiving the bacteria or a processed product thereof or the agent, one can determine that the uremic toxin is reduced by administration of the bacteria or a processed product thereof of the present invention or the agent of the present invention.

Trimethylamine-N-oxide (TMAO) is another uremic toxin. TMAO promotes arteriosclerosis, and has recently been found to serve as a cause of various diseases including acute myocardial infarction, thrombosis, heart failure, and renal failure. When, for example, the TMAO level in a group receiving the bacteria or a processed product thereof of the present invention or the agent of the present invention is lower than that in a group not receiving the bacteria or a processed product thereof or the agent, one can determine that the uremic toxin is reduced by administration of the bacteria or a processed product thereof of the present invention or the agent of the present invention.

Phenyl sulfate (PS) is a representative uremic toxin, and is produced when proteins are broken down by gut bacteria. PS is known to accumulate in the body when renal function is reduced, and its blood level correlates with the incidence of diabetic nephropathy, renal death and the survival rate. Indoxyl sulfate (IS) is another representative uremic toxin. When, for example, the PS level and/or the IS level in a group receiving the bacteria or a processed product thereof of the present invention or the agent of the present invention is lower than that in a group not receiving the bacteria or a processed product thereof or the agent, one can determine that uremic toxins are reduced by administration of the bacteria or a processed product thereof of the present invention or the agent of the present invention.

Creatinine is a uremic toxin that is excreted in urine and then released from the body when the kidney is working properly. When the blood creatinine level is high, reduction in kidney function is suspected. When, for example, the creatinine level in a group receiving the bacteria or a processed product thereof of the present invention or the agent of the present invention is lower than that in a group not receiving the bacteria or a processed product thereof or the agent, one can determine that the uremic toxin is reduced by administration of the bacteria or a processed product thereof of the present invention or the agent of the present invention.

Higher hematocrit and hemoglobin levels indicate thicker blood. When the hematocrit and hemoglobin levels are lower than reference values, the blood is "thin" and the person is prone to anemia. When, for example, the hematocrit and hemoglobin levels in a group receiving the bacteria or a processed product thereof of the present invention or the agent of the present invention are higher than those in a group not receiving the bacteria or a processed product thereof or the agent, one can determine that uremic toxins are reduced by administration of the bacteria or a processed product thereof of the present invention or the agent of the present invention.

The present invention includes aspects in which the configurations as described above are combined in various ways to achieve the effects of the present invention without departing from the technical scope of the present invention.

### Therapeutic or prophylactic agent for autism

The uremic toxins described above also include phenol derivatives including phenyl sulfate (PS) derivatives such as 4-ethylphenyl sulfate. 4-ethylphenyl sulfate has been suggested to be a metabolite related to autism spectrum disorder (ASD) (see, for example, JP 2015-529668 A). Accordingly, when the PS level in a group receiving the bacteria or a processed product thereof of the present invention or the agent of the present invention is lower than that in a group not receiving the bacteria or a processed product thereof or the agent, one can determine that the bacteria or a processed product thereof or the agent is effective for treatment and/or prevention of autism.

### Total cholesterol reducing agent and triglyceride reducing agent for subjects with reduced kidney function

Cholesterol is a lipid, and total cholesterol (total cholesterol level) is a measure of the total amount of cholesterol in the blood. Total cholesterol includes low-density lipoprotein (LDL) and high-density lipoprotein (HDL) . Low-density lipoprotein (LDL), also referred to as bad cholesterol, causes blockages in the blood vessels, and increases the risk of heart disease. High-density lipoprotein (HDL), also referred to as good cholesterol, helps protect us from heart disease. Higher HDL and lower total cholesterol levels are preferred.

Triglyceride is also a lipid, and a high triglyceride level in the blood indicates a great risk for developing arteriosclerosis, fatty liver, hyperlipemia, diabetes mellitus, etc.

The agent of the present invention (in particular, the total cholesterol reducing agent and/or the triglyceride reducing agent for a subject with reduced kidney function) is capable of reducing the total cholesterol level and/or the triglyceride level in a subject with reduced kidney function. Reduction in the total cholesterol level and/or the triglyceride level in a subject with reduced kidney function is useful for, for example, prevention or treatment of a fat-associated disease. The agent of the present invention is also effective for increasing the good cholesterol (HDL) level and/or reducing the bad cholesterol (LDL) level.

The term "reduced kidney function" as used herein specifically refers to the condition where the kidney function of a subject is reduced, and preferably refers to the condition where a subject is suffering from, for example, any one of renal failure, chronic kidney disease, chronic pyelonephritis, acute pyelonephritis, chronic glomerulonephritis, advanced acute nephritic syndrome, nephrotic syndrome, nephrosclerosis, interstitial nephritis, diabetic nephropathy, focal glomerulosclerosis, membranous nephropathy, polycystic kidney syndrome, renovascular hypertension, and hypertension syndrome, and secondary renal diseases associated with the above primary diseases, etc., and more preferably refers to the condition where a subject is suffering from renal failure.

A preferred subject for administration of the total cholesterol reducing agent and/or the triglyceride reducing agent for a subject with reduced kidney function according to the present invention may be, for example, animals, including, for example, but not limited to, humans, rats, mice, rabbits, sheep, pigs, cows, cats, dogs, monkeys, etc.

The preventive or therapeutic effect of the agent of the invention on a fat-associated disease can be confirmed by a known method or a method known per se. For example, when the body weight measurement or the analysis of the amount of liver fat, fat around the epididymis, or the like by, for example, CT scan indicates reduction in the body weight or the amount of fat, one can determined that the agent has preventive or alleviating effect on obesity. In another example, when the pathological analysis of part of liver tissue for examination of lipid droplets and/or fibrosis indicates reduced lipid droplets and/or reduced fibrosis, one can determine that the agent has preventive or therapeutic effect on fatty liver and/or liver fibrosis. In another example, when the analysis of expression of a gene involved in fibrosis of hepatocytes by, for example, quantitative real-time PCR indicates reduced gene expression, one can determined that the agent has preventive or therapeutic effect on liver fibrosis. In another example, when the total cholesterol, ALT (alanine aminotransferase) and AST (aspartate aminotransferase) levels in the plasma are measured and found to be reduced, one can determined that the agent has the effect of maintaining or improving liver function.

### EXAMPLES

The present invention will be described in more detail below with reference to Reference Examples and Examples, but the present invention is not limited thereto. In the following Reference Examples and Examples, reference will be made to specific bacterial strains, but the scope of the present invention is not limited thereto.

### Example 1. Production of bacterial cells

A 0.2 mL frozen stock solution of each bacterial strain (*Lactobacillus gasseri* KD2093, *Bifidobacterium bifidum* G9-1, or *Bacteroides thetaiotaomicron* 6030) was added to 10 mL of GAM liquid medium (GAM broth, Nissui Pharmaceutical Co., Ltd.) supplemented with 0.7% glucose (FUJIFILM Wako Pure Chemical Corporation) and 0.1% polysorbate 80 (Junsei Chemical Co., Ltd.), and the bacterial strain was cultured at 37°C for 24 hours. Then 10 mL of each culture medium was inoculated in 1000 mL of GAM liquid medium supplemented with 0.7% glucose and 0.1% polysorbate 80, and cultured at 37°C for 16 hours. After completion of the culture, the culture medium was centrifuged (at 3,000 × g at 4°C for 10 minutes) . The supernatant was removed and the bacterial cells were washed once with PBS (-). The thus prepared bacterial cells were used for the subsequent experiments. The bacterial cells of *Lactobacillus gasseri* KD2093 were heated at 100°C for 30 minutes to prepare dead bacterial cells for the subsequent experiments.

### Example 2. Administration experiment of bacterial cells to mice with adenine-induced renal failure

(1) Seven-week-old male C57BL/6N Jcl mice (CLEA Japan, Inc.) were fed CE-2 diet (CLEA Japan, Inc.) containing 0.2% adenine for 6 weeks to prepare mice with adenine-induced renal failure (RF) (hereinafter also called "RF mice") . For comparison, male C57BL/6N Jcl mice at the same age (seven weeks old) were fed normal CE-2 diet (not containing adenine) for 6 weeks to prepare normal mice without renal failure.
(2) The bacterial cells prepared in Example 1 (*Lactobacillus gasseri* KD2093, dead bacterial cells of *Lactobacillus gasseri* KD2093, *Bifidobacterium bifidum* G9-1, or *Bacteroides thetaiotaomicron* 6030) or distilled water (dw) as a control was orally administered to RF mice prepared in the above (1) once a day consecutively for two weeks.

The feeding schedule of the diet and the details of the experimental groups are as shown in Fig. 2. Each group contained n = 6 mice. After the start of administration of the bacterial cells, RF mice were fed CE-2 diet containing 0.2% adenine for one week, and after the first week of administration of the bacterial cells, CE-2 diet containing 0.2% adenine was switched to normal CE-2 diet (not containing adenine) for one week to eliminate the possibility that the administered bacterial cells may inhibit absorption of adenine in the diet and improve kidney function. On the following day after the two-week administration of the bacterial cells, the mice were dissected and blood samples were collected.

### Example 3. Measurement of uremic toxins

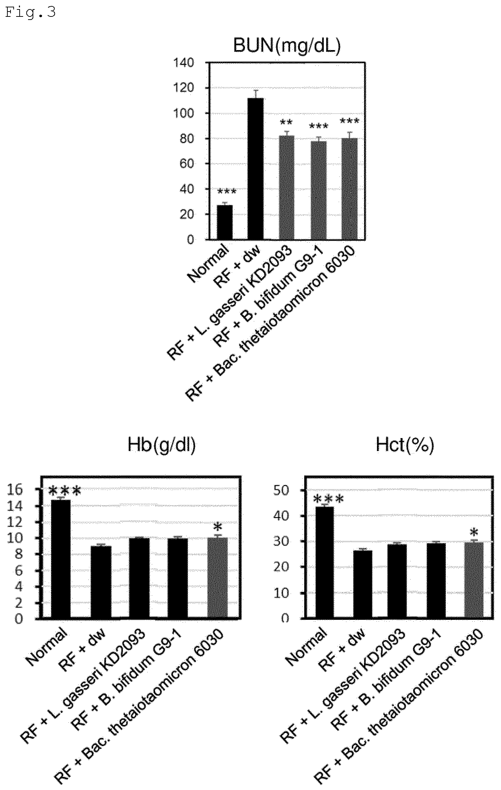

The concentrations of uremic toxins in the blood samples collected from the normal mice and RF mice in Examples 2 (RF + dw, RF + *L. gasseri* KD2093, RF + *B. bifidum* G9-1, and RF + *Bac. thetaiotaomicron* 6030) were measured. The results are shown in Figs. 3 to 6. The concentrations of uremic toxins other than BUN, i.e., creatinine, 1-methyladenosine (m1A), trimethylamine-N-oxide (TMAO), phenyl sulfate (PS) and indoxyl sulfate (IS) were measured by LC-MS. BUN, hematocrit and hemoglobin levels were measured using i-STAT cartridges (Abbott) .

### Summary of measurement results of uremic toxins

The results shown in Fig. 3 demonstrate that the blood urea nitrogen (BUN) levels in RF mice that received the bacterial cells of the present invention (RF + *L. gasseri* KD2093, RF + *B. bifidum* G9-1, and RF + *Bac. thetaiotaomicron* 6030) were reduced by as much as about 30% as compared with control RF mice that received distilled water (RF + dw). The hematocrit and hemoglobin levels were also significantly improved in mice that received *Bac. thetaiotaomicron* 6030, and the values were also improved in mice that received *L. gasseri* KD2093 or *B. bifidum* G9-1.

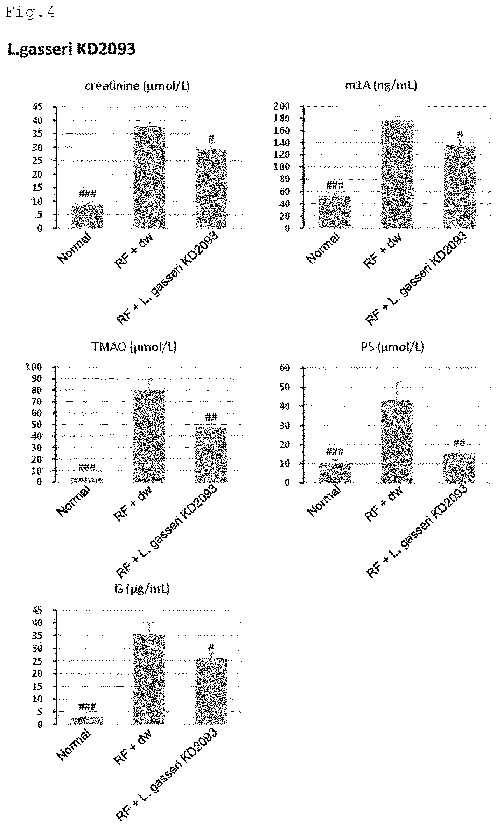

The results shown in Fig. 4 demonstrate that RF mice that received *L. gasseri* KD2093 of the present invention showed improvement in all of uremic toxins, including creatinine, 1-methyladenosine (m1A), trimethylamine-N-oxide (TMAO), phenyl sulfate (PS) and indoxyl sulfate (IS), as compared with the control. *L. gasseri* KD2093 showed higher effect of reducing TMAO and PS.

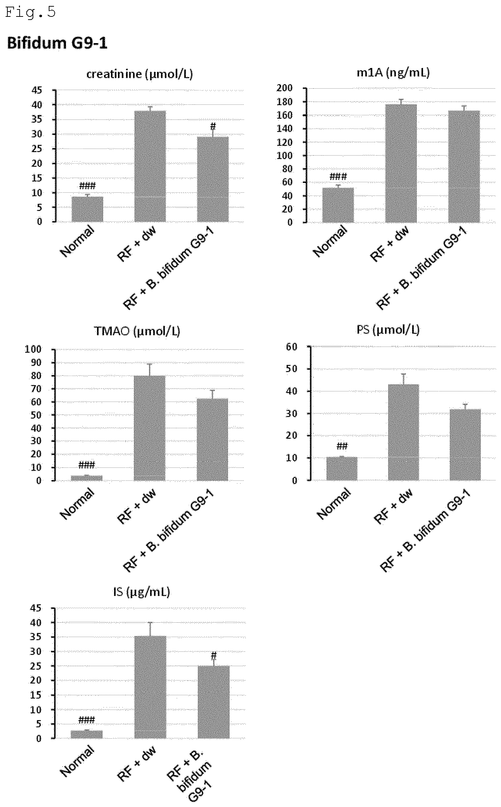

The results shown in Fig. 5 demonstrate that RF mice that received *B. bifidum* G9-1 of the present invention showed improvement in all of uremic toxins, including creatinine, m1A, TMAO, PS and IS, as compared with the control. *B. bifidum* G9-1 showed higher effect of reducing creatinine and IS.

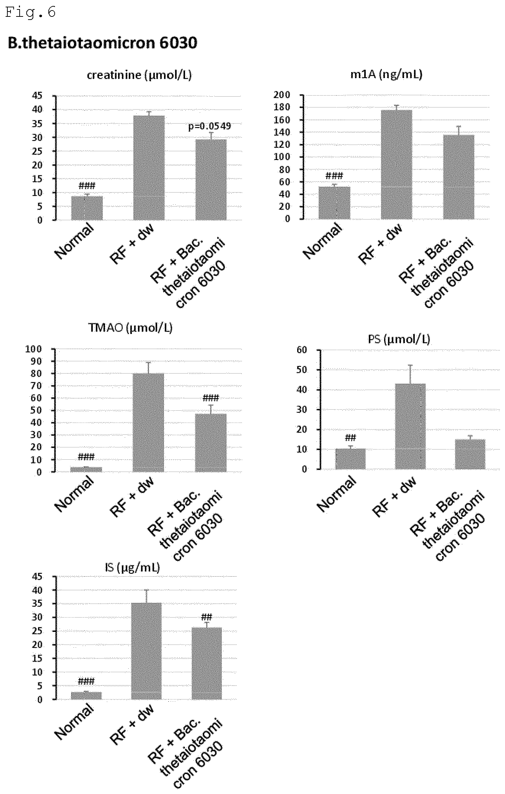

The results shown in Fig. 6 demonstrate that RF mice that received *Bac. thetaiotaomicron* 6030 of the present invention showed improvement in all of uremic toxins, including creatinine, m1A, TMAO, PS and IS, as compared with the control. *Bac. thetaiotaomicron* 6030 showed higher effect of reducing creatinine, TMAO and PS.

### Measurement of total cholesterol (T-Cho (TC)) and triglyceride (TG)

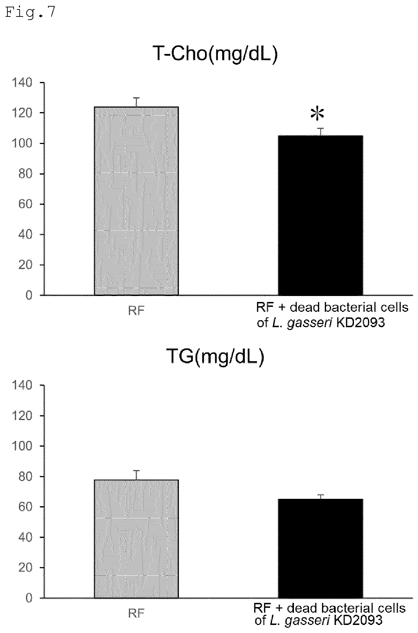

TC and TG levels in the blood samples collected from RF mice of Example 2 (RF + dw, and RF + dead bacterial cells of *L. gasseri* KD2093; mice with reduced kidney function) were measured. The results are shown in Fig. 7. The measurement of TC and TG was performed on FUJI DRI-CHEM 7000 V using 10 µL of plasma samples.

### Summary of measurement results of TG and TC

The results shown in Fig. 7 demonstrate that the T-Cho (TC) and TG levels in RF mice that received the bacterial cells of the present invention (RF + dead bacterial cells of *L. gasseri* KD2093) were reduced as compared with control RF mice that received distilled water (RF + dw). The dead bacterial cells of *L. gasseri* KD2093 showed higher effect of reducing T-Cho (TC) .

### INDUSTRIAL APPLICABILITY

The method according to the present invention is capable of efficiently producing a uremic toxin reducing agent, a total cholesterol reducing agent and a triglyceride reducing agent in an industrially advantageous manner, and is therefore useful.

## Claims

1. A uremic toxin reducing agent comprising any one of the following bacteria (a) to (c):
(a) one or more species of *Lactobacillus* bacteria selected from the group consisting of *Lactobacillus gasseri, Lactobacillus johnsonii* and *Lactobacillus casei,*
(b) one or more species of *Bifidobacterium* bacteria selected from the group consisting of *Bifidobacterium longum, Bifidobacterium bifidum, Bifidobacterium breve* and *Bifidobacterium infantis,* or
(c) one or more species of *Bacteroides* bacteria.

2. The uremic toxin reducing agent according to claim 1, wherein the one or more species of *Lactobacillus* bacteria are *Lactobacillus gasseri* KD2093 (Accession No. NITE BP-02913).

3. The uremic toxin reducing agent according to claim 1, wherein the one or more species of *Bifidobacterium* bacteria are *Bifidobacterium bifidum* G9-1 (Accession No. NITE BP-817).

4. The uremic toxin reducing agent according to claim 1, wherein the one or more species of *Bacteroides* bacteria are *Bacteroides thetaiotaomicron.*

5. The uremic toxin reducing agent according to any one of claims 1 to 4, wherein a uremic toxin to be reduced is one or more selected from the group consisting of blood urea nitrogen (BUN), 1-methyladenosine (m1A), trimethylamine-N-oxide (TMAO), phenyl sulfate (PS), indoxyl sulfate (IS) and 4-ethylphenyl sulfate (4-EPS).

6. The uremic toxin reducing agent according to any one of claims 1 to 5, wherein the agent further reduces a creatinine level.

7. The uremic toxin reducing agent according to any one of claims 1 to 6, wherein the agent further increases a hematocrit level and/or a hemoglobin level.

8. A method for producing the uremic toxin reducing agent according to any one of claims 1 to 7, comprising culturing any one of the bacteria as described in claim 1, and collecting a culture product.

9. Use of any one of the bacteria as described in claim 1 in the production of a uremic toxin reducing agent.

10. A total cholesterol reducing agent for a subject with reduced kidney function, comprising any one of the following bacteria (a) to (c):
(a) one or more species of *Lactobacillus* bacteria selected from the group consisting of *Lactobacillus gasseri, Lactobacillus johnsonii* and *Lactobacillus casei,*
(b) one or more species of *Bifidobacterium* bacteria selected from the group consisting of *Bifidobacterium longum, Bifidobacterium bifidum, Bifidobacterium breve* and *Bifidobacterium infantis,* or
(c) one or more species of *Bacteroides* bacteria.

11. A triglyceride reducing agent for a subject with reduced kidney function, comprising any one of the following bacteria (a) to (c):
(a) one or more species of *Lactobacillus* bacteria selected from the group consisting of *Lactobacillus gasseri, Lactobacillus johnsonii* and *Lactobacillus casei,*
(b) one or more species of *Bifidobacterium* bacteria selected from the group consisting of *Bifidobacterium longum, Bifidobacterium bifidum, Bifidobacterium breve* and *Bifidobacterium infantis,* or
(c) one or more species of *Bacteroides* bacteria.
